# EUROPEAN PATENT APPLICATION

(11) **EP 4 506 066 A1**
(43) Date of publication of application: **12.02.2025**
(21) Application number: 23191136.3
(22) Date of filing: 11.08.2023
(51) Int. Cl.: B05B 17/06, B05B 9/04, B05B 7/00, A61L 2/10, A63J 5/02

(54) **HAZE GENERATING DEVICE**

(71) Applicant: Harman Professional Denmark ApS, 8200 Aarhus N (DK)
(72) Inventor: SCULLY, Nicholas John, Horncastle LN9 5EZ (GB)
(74) Representative: Kraus & Lederer PartGmbB

(57) **Abstract**

The present application relates to a haze generating device (100) for generating aerosol from a liquid. The haze generating device (100) comprises at least one chamber (102-116) in which the aerosol is generated. Each chamber (102-116) comprises a chamber housing including a bottom and a side wall extending from the bottom to a top of the chamber (102-116), an ultrasonic transducer (122-136) arranged at the bottom, and a filter element (502, 504) arranged at the top of the chamber (102-116). In operation of the haze generating device (100), the chamber (102-116) is at least partially filled with the liquid to a filling level (802) such that at least an ultrasound emitting surface (804, 806) of the ultrasonic transducer (122-136) is immersed in the liquid. The chamber (102-116) has a directed gas inlet (808, 810) at the side wall configured to direct a gas stream introduced into the chamber (102-116) in a direction substantially parallel (812, 814) to a surface of the liquid in the chamber (102-116) and substantially along the side wall. The filter element (502, 504) comprises a plurality of guide surfaces (702) configured to direct the gas stream out of the chamber (102-116) in a direction substantially perpendicular (828, 830) to the surface of the liquid in the chamber (102-116).

## Description

### Technical Field

The present application relates to a haze generating device for generating aerosol from a liquid. The haze generating device comprises at least one chamber in which the aerosol is generated by means of an ultrasonic transducer.

### Background

A haze generating device, also called haze machine, haze generator or simply hazer, is a type of device used to produce the effect of a dreamy or mysterious atmosphere in a live show or space. It typically produces an extremely fine mist similar to that of a fog machine but at lower temperatures and with fewer emissions. Its primary function is to disperse light differently, reflecting the light emitted from light sources back into the audience or room, creating a more significant visual impact and a feeling of depth. Many popular shows and clubs use haze machines to enhance the atmosphere and emphasize certain elements of their production. The haze can also be used to filter out any bright colours and shapes, making them more subtle.

A haze machine may take in a flow of air from the atmosphere and may pass it through a vaporizer that vaporizes a liquid or fogging solution stored in the machine's reservoir. The air then dissipates the fogging solution into a vaporized mist of tiny water droplets, which is then blown into the air. There are several techniques for vaporizing the liquid in a haze machine. These include atomizing and nebulizing the liquid by means of a spray pump into the air. There are also ultrasonic and thermal methods that rely on sound and temperature respectively to vaporize the liquid. Ultrasonic haze machines use transducers submerged in liquid to vaporize the liquid and to create the haze. Ultrasonic haze machine may generate their effects with much less noise than other technologies.

Generally, using ultrasound, it is easy to mix two materials that are not soluble in each other. Such a mixture is generally called a colloid or dispersion. If the mixture consists of small droplets of liquid, it is called an aerosol (or fog) if these droplets are dispersed in a gas, and an emulsion if they are in another liquid.

One of the main applications of ultrasonic dispersion is the atomization of liquids, i.e. the production of aerosols. For this purpose, ultrasonic waves can be used that arrive from below the surface of the liquid. If the sonic intensity is high enough, a spray of droplets or fog will emerge from the surface as a side effect of the formation of an ultrasonic fountain. It should be noted that the aerosol produced is a monodispersion, i.e. the liquid droplets are virtually uniform in size. This type of fog formation is not a thermal process, but capillary waves are excited on the liquid surface from which the droplets are propelled into the air.

For the generation of a capillary wave, the propagation of surface waves on a liquid can be considered. If the surface is subjected to a harmonic perturbation with a certain angular frequency, a potential flow will develop in the liquid that describes a surface wave propagating in the horizontal direction with an amplitude that decreases with increasing depth of the liquid, i.e. in the downward vertical direction. This gives rise to gravitational waves in combination with capillary waves. Assuming that a liquid as a whole is moved sinusoidally up and down, it will experience an alternating acceleration superimposed on the gravitational acceleration. This modulation of the gravitational acceleration can be achieved by an ultrasonic wave propagating to the surface. The resulting wave has a sinusoidally varying restoring force and certain inherent instabilities that lead to the spontaneous excitation of a standing capillary wave. At low sound intensities, the capillary waves are sinusoidal. As the ultrasonic amplitude increases, the amplitude of the capillary waves also increases, and their shape becomes increasingly different from a sine wave due to nonlinearities. Finally, at high amplitude, droplets are propelled from the crest of the wave, resulting in atomization of the liquid. The droplets may be uniform in size because their diameters are expected to be a certain fraction of the capillary wavelength. The ultrasound can be generated by a piezoceramic transducer operating at 0.5 - 2 MHz and producing a particularly high sound intensity at the surface of the liquid. For example, a droplet diameter of 2 µm can be obtained at an ultrasonic frequency of 1.8 MHz.

Critical aspects of the design of a haze generating device may relate to high and continuous quality of the generated haze, liquid consumption, cooling of the ultrasonic transducers, cooling of the liquid, control of bacterial growth in the liquid, and robustness of the haze generating device during operation and transportation.

### Summary

In view of the above, there is a need for improvements in haze generating devices that address at least some of the above mentioned aspects.

According to the present application, this need is met by the features defined in the independent claim. The dependent claims define embodiments.

A haze generating device for generating aerosol from a liquid includes at least one chamber in which the aerosol is generated. For example, the haze generating device may include two to twenty chambers depending, on the amount of haze to be generated in a given period of time and depending on a size or volume of the venue. The chambers may be arranged in an array or matrix. In various examples, eight chambers may be arranged in an array of four by two chambers. Thus, a compact design of the haze generating device and can be achieved for installation and transportation.

Each chamber of the at least one chamber includes a chamber housing. The chamber housing has a bottom and a side wall extending from the bottom to a top of the chamber. For example, each chamber may have a cuboidal shape. At the bottom of each chamber, an ultrasonic transducer is arranged. A filter element is arranged at the top of each chamber. In operation of the haze generating device, the chamber is at least partially filled with the liquid to a filling level such that at least the ultrasound emitting surface of the ultrasonic transducer is immersed in the liquid. The ultrasound emitting surface of the ultrasonic transducer may be arranged horizontally, i.e. substantially in parallel to the surface of the liquid in the chamber, or the ultrasound emitting surface of the ultrasonic transducer may be inclined with respect to the surface of the liquid in the chamber, for example at an angle between 15 and 22 degrees.

Each chamber has a directed gas inlet at the side wall configured to direct a gas stream introduced into the chamber in a direction substantially parallel to a surface of the liquid in the chamber and substantially along the side wall, i.e. in parallel or tangential to the side wall. As a result, a circular flow of the gas in a plane and parallel to the surface of the liquid within the chamber can be achieved.

The filter element has a plurality of guide surfaces configured to direct the gas stream out of the chamber in a direction substantially perpendicular to the surface of the liquid in the chamber. Circulating the gas above the surface of the liquid and then directing the gas vertically out of the chamber may contribute to continuously generate a homogeneous aerosol. In particular, emission of larger droplets may be prohibited. The haze can be produced in a high and continuous quality.

In various examples, each guide surface of the plurality of guide surfaces of the filter has a first surface portion extending perpendicularly to the surface of the liquid in the chamber and a second surface portion extending at an angle from the first surface portion toward the surface of the liquid in the chamber. For example, the second surface portion may extend downwardly from the first surface portion at an angle in a range of 90 to 110 degrees, preferably 100 degrees. The first surface portion and the second surface portion may be of similar size. For example, a length of extension of the second surface portion away from the first surface portion may be in a range of 20% to 150% of the length of extension of the first surface portion perpendicular to the surface of the liquid in the chamber. In some examples, the length of extension of the second surface portion away from the first surface portion may be the same as the length of extension of the first surface portion perpendicular to the surface of the liquid in the chamber. The angled guide surfaces may separate the large droplets from the aerosol, for example droplets which are larger than 2 or 3 µm in diameter, and may reliably return the separated large droplets to the liquid for reuse. The slightly downward tilted second surface portion assists in the rapid return of the separated large droplets to the liquid and prevents clogging of the filter. Liquid consumption can be reduced.

The first and second surface portions may each be planar. The second surface portion may extend from an edge of the first surface portion. The first surface portions of the plurality of guide surfaces may be arranged parallel to each other with a gap therebetween. A width of the gap may be a few millimetres, e.g. 2 to 10 mm, preferably 3 to 5 mm. In addition or as an alternative, the second surface portions of the plurality of guide surfaces may be arranged parallel to each other. Gaps may be provided between the second surface portions. A width of these gaps may be a few millimetres, e.g. 2 to 5 mm. For example, the angled guide surfaces may be arranged in the staggered manner spaced from each other to enable a compact design of the filter. The circular gas stream above the surface of the liquid in the chamber is received by the second surface portions and guided along the angled connection between the second surface portions and the first surface portions in the vertical direction upwards out of the filter. A uniform and dense aerosol may be achieved.

According to the various examples, the haze generating device includes a reservoir or tank for the liquid and a pump coupled between the reservoir and the chambers for pumping the liquid from the reservoir into the chambers. In addition, a return duct may be included which connects a liquid outlet of each of the the chambers to the reservoir. The liquid outlet may be located at a level corresponding to the filling level. During operation of the haze generating device, the pump may pump the liquid from the reservoir into the chambers. When the level of liquid in the chambers reaches the level of the liquid outlet, the liquid may flow back through the liquid outlet and the return duct into the reservoir. This allows the level to be maintained automatically at the level of the liquid outlet without the need for additional level sensors. In addition, the liquid is continuously circulated so that the liquid heated in the chamber by the ultrasonic transducers is continuously replaced by cool liquid from the reservoir. In this way, the ultrasonic transducers as well as the fluid may be cooled.

In some examples, the haze generating device includes two or more chambers. In this case, each chamber may have an individual liquid outlet. The filling level may be maintained automatically in each chamber without providing any additional level sensor.

In some further examples, the haze generating device includes a plurality of groups of chambers. Each group of chambers is fluidically separated from the other groups of chambers.

For example, the haze generating device includes four groups of two chambers. Each group of chambers has an individual liquid outlet. The filling level may be maintained automatically in each chamber group without providing any additional level sensor. Such a modular chamber design allows the haze generating device to operate in not accurately aligned arrangements, for example up to +/- 15 degrees from the horizontal in pitch and +/- 5 degrees in roll.

In various examples, a sensor, such as an optical liquid sensor, may be provided in the return duct for monitoring a return flow of the liquid from the chambers to the reservoir. Liquid circulation can be monitored and a warning can be output in case of a failure. Fault causes may be an empty reservoir, a clogged duct, a pump failure or an incorrect arrangement of the haze generating device, for example in an excessively tilted position. In some examples, a further pump may be provided in the return duct for pumping the liquid from the chamber into the reservoir. The further pump may be controlled depending on a sensor value from the sensor. The further pump may enable a more flexible arrangement of the return duct and the reservoir with respect to the chambers. For example, the further pump may allow the liquid to be pumped the from the chamber into the reservoir even if parts of the return duct or the filling level of the liquid in the reservoir is above the level of the liquid outlet of the chamber.

The haze generating device may further include an ultraviolet light source for irradiating the liquid with ultraviolet light. For example, the ultraviolet light source may be provided in the reservoir emitting ultraviolet light into the liquid in the reservoir. In further examples, the ultraviolet light source may be provided in the return duct emitting ultraviolet light into the liquid flowing through the return duct. When the gas passing along the surface of the liquid in the chamber and through the filter is ambient air drawn into the haze generating device by fans, this air may contaminate the liquid with airborne organic material leading to fungal and bacterial growth in the reservoir, ducts, pumps and/or chambers. Such fungal and bacterial growth may be prevented by irradiating the liquid with ultraviolet light,.

According to various examples, the reservoir may be provided with a filling inlet for filling and refilling liquid into the reservoir. The reservoir may be configured such that it provides a level control ensuring that a predefined volume within the reservoir cannot be filled with the liquid via the filling inlet. Thus, although the predefined volume of the reservoir is currently not filled with liquid, the configuration of the reservoir prohibits to fill this predefined volume via the filling inlet. The predefined volume may be considered as a "hidden volume" which is not usable for the liquid from the point of view of a user or operator filling or refilling the reservoir. For example, the filling inlet of the reservoir may include a vertical tube extending downward within the reservoir. A valve arrangement may control venting of the "hidden volume". The valve arrangement may be controlled by a control circuit of the haze generating device. In some examples, a valve ball may be provided at the lower end of the vertical tube. When the liquid level exceeds the lower end of the tube, the valve ball closes the tube so that no further liquid can be introduced into the reservoir, although there is unfilled volume space along the vertical tube.

The predefined "hidden volume" may correspond to a sum of the volumes of the liquid in the plurality of chambers during operation of the haze generating device. The haze generating device may include a control circuit configured to operate the pump in the reverse direction to pump the liquid from the chambers back into the reservoir. Since the configuration of the reservoir guarantees that the predefined "hidden volume" cannot be filled by an operator via the filling inlet, it can be ensured that the reservoir provides a sufficient volume to receive the liquid from the chambers. The liquid may be pumped from the chambers back to the reservoir for transportation of the haze generating device to ensure that no liquid from the chambers can leak from the haze generating device during transport even if the haze generating device is tilted or turned upside down. The control circuit may include an electrical energy storage means, for example a rechargeable battery or a supercapacitor, for operating the pump in the reverse direction using electrical energy from the electrical energy storage means when it is detected that an external power supply is disconnected. Thus, the chambers are automatically emptied the haze generating device is switched off or disconnected from the power supply so that the haze generating device is ready for transport.

In various examples, the haze generating device includes an additive tank and a mixing device coupled to the additive tank and the reservoir. The mixing device is configured to add an additive or concentrate contained in the additive tank to the liquid in the reservoir. Glycol or glycerine may be used as an additive to be mixed with tap water. The concentration of the additive in the tap water may be in the range of 5 to 10%, for example 8%. Thus, only a small amount of additive needs to be transported to the venue, while the tap water may be easily available locally. A good mixture of the additive and the tap water can be achieved by circulating the liquid through the chambers and the reservoir as described above.

In various examples, the ultrasonic transducer is driven by a control circuit with an electrical signal having a sinusoidal waveform. For example, each ultrasonic transducer of the plurality of chambers may be driven individually or groups of ultrasonic transducers may be driven collectively. Each ultrasonic transducer may be controlled by the control circuit in parallel resonance. For example, the ultrasonic transducer may represent the inductance of an LC circuit and may be operated at or near resonant frequency of this LC circuit. In this way, a power efficient operation of the ultrasonic transducer may be achieved. Further tuning of the sinusoidal waveform for driving the ultrasonic transducer to maintain the ultrasonic transducer in parallel resonance may be performed in response to an electrical noise signal determined at an electrical current driving the ultrasonic transducer.

If the haze generating device includes two or more chambers, the control circuit can control the operation of the two or more ultrasonic transducers to balance the operating times of the ultrasonic transducers. For example, depending on the required haze output, not all chambers may need to be active at the same time. In order to equalise the operating times in such scenarios, the control circuit can switch the ultrasonic transducers on and off according to a predefined scheme in which for a first period of time a first group of ultrasonic transducers is activated while others are deactivated, for a second period of time a second group of ultrasonic transducers is activated while others are deactivated, for a third period of time a third group of ultrasonic transducers is activated while others are deactivated and so on until each ultrasonic transducer has been activated at least once and then the scheme is repeated. In this way, even wear of the ultrasonic transducers can be achieved.

The features set out above and those described below may be used not only in the corresponding combinations explicitly set out, but also in other combinations or in isolation, without departing from the scope of protection of the present invention.

### Brief description of the Drawings

The present invention will now be described more fully hereinafter with reference to the accompanying drawings, in which embodiments of the invention are shown. However, this invention should not be construed being limited to the embodiments set forth herein. Rather, these embodiments are provided so that this disclosure will be thorough and complete, and will fully convey the scope of the invention to those skilled in the art. Like numbers refer to like elements throughout.
FIG. 1 schematically shows an isometric view of a haze generating device according to one of a number of embodiments;
FIG. 2 schematically shows a further isometric view of the haze generating device of FIG. 1;
FIG. 3 schematically shows a side view of the haze generating device of FIG. 1;
FIG. 4 schematically shows a further side view of the haze generating device of FIG. 1;
FIG. 5 schematically shows an isometric view of a pair of chambers of the haze generating device of FIG. 1;
FIG. 6 schematically shows an isometric exploded view of the pair of chambers of FIG. 5;
FIG. 7 schematically shows a top view of the pair of chambers of FIG. 5;
FIG. 8 schematically shows a sectional view of the pair of chambers of FIG. 5;
FIG. 9 schematically shows a control architecture and liquid control of the haze generating device of FIG. 1;
FIG. 10 shows method steps performed by a control circuit of the haze generating device of FIG. 1;
FIG. 11 schematically shows a perspective view of a reservoir according to one of a number of embodiments;
FIG. 12 schematically shows a top view of the reservoir of FIG. 11;
FIG. 13 schematically shows a sectional view of the reservoir of FIG. 11;
FIG. 14 schematically shows a water tank according to one of a number of embodiments.

### Detailed description

The properties, features and advantages of this invention described above and the way in which they are achieved will become clearer and more clearly understood in association with the following description of the exemplary embodiments which are explained in greater detail in connection with the drawings. For simplicity and illustrative purposes, the present invention is described by referring mainly to an exemplary embodiment thereof. In the following description, numerous specific details are set forth in order to provide a thorough understanding of the present invention. However, it will be readily apparent to one of ordinary skill in the art that the present invention may be practiced without limitation to these specific details. In this description, well known methods and structures have not been described in detail so as not to unnecessarily obscure the present invention.

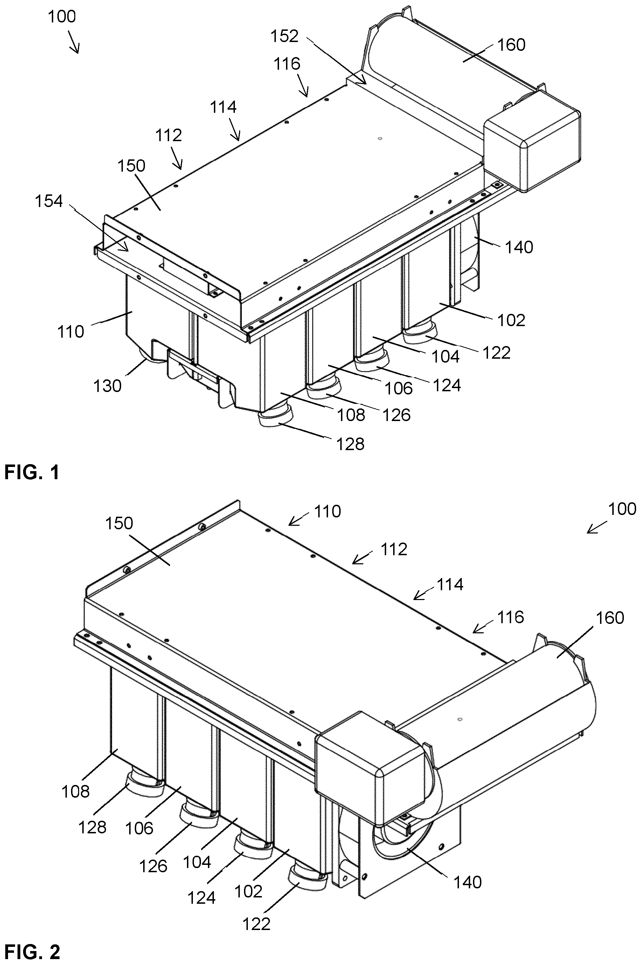

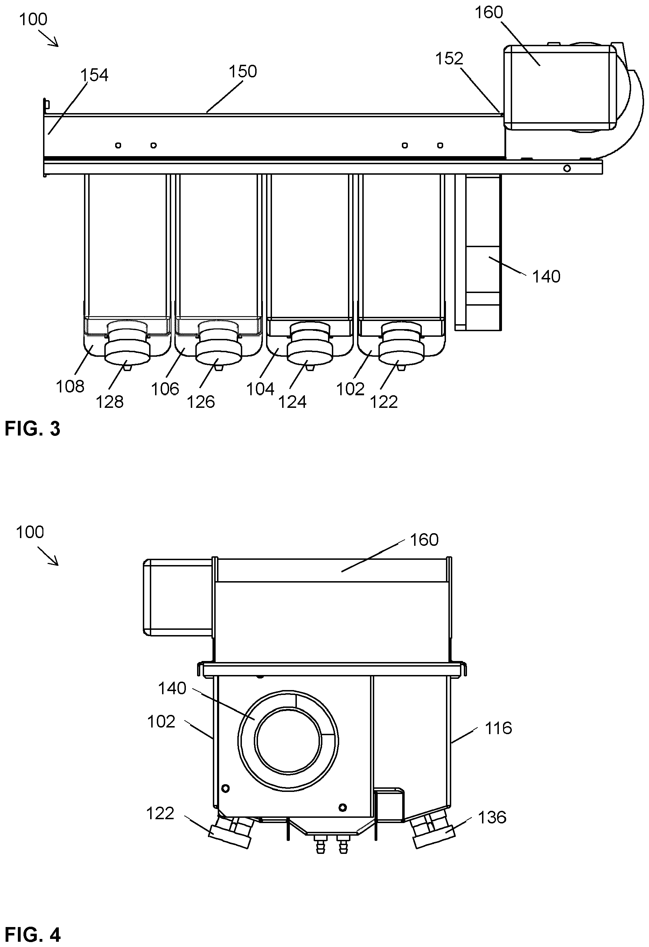

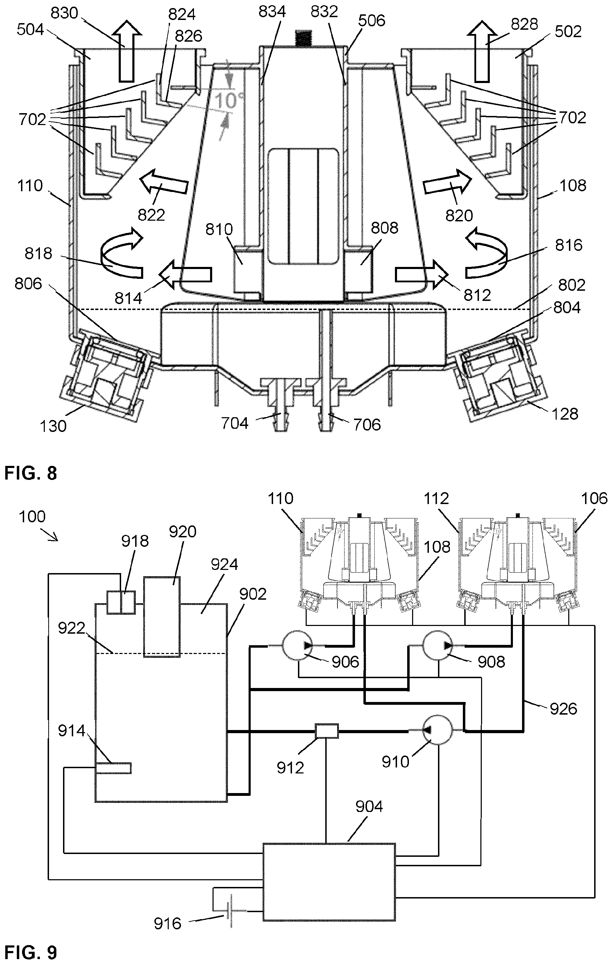

FIG. 1 and FIG. 2 schematically show isometric views of a haze generating device 100 from different directions. FIG. 3 and FIG. 4 show side views of the haze generating device 100 from different directions. In operation, the haze generating device 100 may generate aerosols from a liquid. The liquid may be provided in a reservoir which is not shown in FIGs. 1-4 and which will be described below in connection with FIG. 9. The haze generating device 100 includes a plurality of chambers. The haze generating device 100 may have any number of chambers, for example in a range from 1 to 20. The haze generating device 100 shown in FIGs. 1-4 has eight chambers which are arranged in pairs, i.e. the eight chambers are arranged in a matrix of 4 x 2 chambers. In FIG. 1 only five of the eight chambers are visible, i.e. chambers 102, 104, 106, 108 and 110 are visible and chambers 112, 114 and 116 are not visible. In the side view of FIG. 4, the chambers 102 and 116 are visible. The pairs of chambers, i.e. chamber pair 102/116, chamber pair 104/114, chamber pair 106/112 and chamber pair 108/110, may have essentially the same structure. The chamber pairs are arranged in a row. At the bottom of each chamber, a corresponding ultrasonic transducer 122-136 is arranged. Again, in FIG. 1 only the transducers 122, 124, 126, 128 and 130 of the chambers 102, 104, 106, 108 and 110 are visible, and in FIG. 4 the transducers 122 and 136 of chambers 102 and 116 are visible.

In operation of the haze generating device 100, each chamber 102-116 is filled with liquid from the reservoir such that ultrasound emitting surfaces of the transducers 122-136 are immersed in the liquid. When the ultrasonic transducers are driven, for example with a sinusoidal waveform in a range of 1 to 2 MHz, droplets are generated on the liquid surface in the chambers 102-116.

At one end of the row of chamber pairs, a fan 140 is arranged which blows ambient air into the chambers 102-116 as will be described below in more detail. The droplets generated in the chambers 102-116 are received by the stream of air from the fan 140 and are blown out together with the air at an upper end of each chamber 102-116 as will be described in more detail below. Thus, an aerosol is generated in the chambers 102-116 and exhausted from the chambers 102-116. A cover 150 is provided over the upper ends of the chambers 102-116. The cover 150 forms a channel. At one end 152 of the cover 150 a further fan 160 is provided which blows ambient air into the channel. The aerosol in combination with the ambient air from the further fan 160 forms the haze which is discharged at the opposite other end 154 of the cover 150.

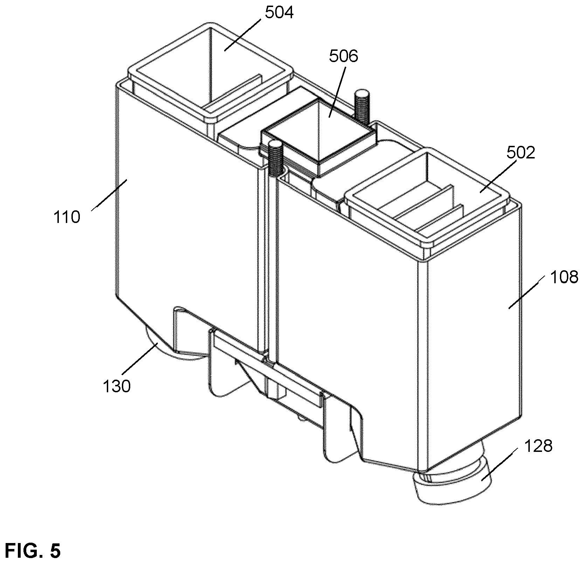

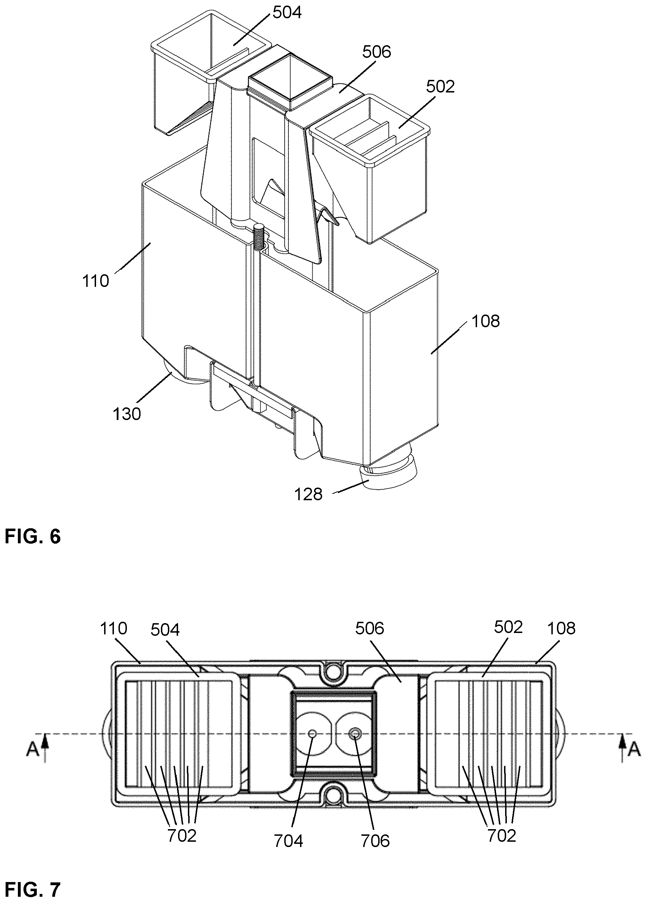

FIG. 5 schematically shows an isometric view of a chamber pair, for example the chamber pair 108/110. As explained above, corresponding ultrasonic transducers 128 and 130 are arranged at a bottom side of each chamber 108, 110. The chambers 108, 110 are open at the top sides, see also the partially exploded view in FIG. 6. A corresponding filter element 502, 504 is provided at the top side of each chamber. Furthermore, an inlet structure 506 is provided which directs the airflow from the fan 140 into each chamber 108, 110. As can be seen in the top view of the chamber pair 108/110 in FIG. 7, each filter element 502, 504 is provided with a plurality of guide surfaces 702. Also, as shown in FIG. 7, a liquid inlet 704 and a liquid outlet 706 are provided in the chamber pair 108/110.

FIG. 8 shows a sectional view along section A-A of FIG. 7. In operation of the haze generating device 100, liquid is pumped into the chambers 108, 110 via the liquid inlet 704. The liquid may be a mixture of water and an additive, for example glycerine or glycol, for example 5% to 15% by weight or volume. The liquid outlet 706 is at the higher level than the liquid inlet 704. When the liquid level in the chambers 108, 110 reaches a required filling level 802, any excess of liquid may flow out of the chambers 108, 110 via the liquid outlet 706. Thus, no specific sensor detection is required to control the level of the liquid in the chambers 108, 110. When the filling level 802 is reached, the ultrasound emitting surfaces 804, 806 of the ultrasonic transducers 128 and 130 are immersed in the liquid. In operation of the ultrasonic transducers 128 and 130, droplets are generated that separate from the liquid surface above the filling level 802.

Further in operation, the air from the fan 140 is introduced into each chamber 108, 110 via the inlet structure 506. The inlet structure 506 provides for each chamber 108, 110 a respective gas inlet 808 and 810 which introduces the air from the fan 140 in the horizontal direction, i.e. parallel to the surface of the liquid in the chamber, above the filling level 802 along a side wall of the chamber 108, 110, for example along the side wall extending outwardly from the centre between the pair of chambers 108/110. As shown in FIG. 8, in the chamber 108 the air may be directed horizontally to the right in the direction of arrow 812, and in the chamber 110 the air may be directed horizontally to the left in the direction of arrow 814. Within each chamber 108, 110, the airflow may then begin to circulate within the respective chamber 108, 110 in a horizontal plane as indicated by arrows 816 and 818. The circulating air may pick up the droplets from the liquid, thereby generating an aerosol. The circulating aerosol may ascend within the chambers 108, 110 to the respective filters 502, 504, see arrows 820 and 822.

Each filter 502, 504 has a plurality of guide surfaces 702 or baffles. In the example shown in FIG. 8, each filter 502, 504 has five guide surfaces 702. However, the filters 502, 504 may have any other number of guide surfaces, for example in a range from 3 to 10. Each guide surface 702 has a first surface portion 824 in the vertical direction, i.e. perpendicular to the filling level 802, and a second surface portion 826 extending from the lower edge of the first surface portion 824 in the lateral direction such that an angle between the first surface portion 824 and the second surface portion 826 is in a range of, for example, 90 to 110 degrees. In the example shown in FIG. 8, the first surface portion 824 and the second surface portion 826 include an angle of about 100 degrees such that the second surface portion 826 is slightly inclined downwardly by a 10 degrees with respect to the horizontal.

The five guide surfaces 702 are separated from each other, i.e. there are gaps between each two neighbouring guide surfaces 702. The gaps may have a width of a few millimetres, for example in a range of 2 to 10 millimetres, for example 3 or 5 millimetres. The first surface portions 824 of the guide surfaces 702 may be parallel to each other. Likewise, the second surface portions 826 may be parallel to each other. The guide surfaces 702 may be stacked or staggered as shown in FIG. 8, i.e. a section of the first surface portion 824 of one guide surface 702 partially overlaps the first surface portion 824 of the neighbouring guide surface 702, and/or a section of the second surface portion 826 of one guide surface 702 partially overlaps the second surface portion of the neighbouring guide surface 702.

The ascending circulating aerosol enters the gaps between the second surface portions 826 and is deflected in the vertical direction by the first surface portions 824 and exits the filter 502, 504 in the vertical direction as indicated by arrows 828 and 830. As a result of the deflection within the filter 502, 504, droplets having a diameter greater than a desired diameter, for example 1 to 2 micrometres may collide with the guide surfaces 702, from which they may flow down by gravity along the first and second surface portions 824, 826 back to the liquid in the chamber 108, 110. As a result, a homogeneous aerosol is obtained at the outlet of the filter 502, 504 and liquid of droplets having an unwanted size can be reused.

As depicted in FIG. 8, the ultrasound emitting surfaces 804, 806 of the transducers 128, 130 are inclined with respect to the horizontal, i.e. with respect to the surface of the liquid. It has been found that inclining the ultrasound emitting surfaces 804, 806 by an angle in the range from 15 to 22 degrees, preferably by an angle of 18 degrees, results in efficient generation of droplets. In addition, droplets of the ultrasonic fountain generated by the ultrasonic transducers 128, 130 may fly upwards towards upper wall sections 832, 834 of the inlet structure 506. Larger droplets may collide with these upper wall sections 832, 834 and may flow back down for reuse, while smaller droplets are pulled along with the airflow 808-822. This may prevent formation of larger droplets. Additionally, by directing the airflow along the first surface portions 826 and the second surface portions 824 may further separate larger droplets. The aerosol generated in the chambers 102-116 is discharged by the respective filters into the channel provided by the cover 150 and blown out by the fan 160 as discussed above in connection with FIG. 1.

FIG. 9 schematically shows further components of the haze generating device 100. As shown, the haze generating device 100 includes a plurality of pairs of chambers, for example chamber pair 108/110 and chamber pair 106/112, a reservoir 902 for the liquid, a control circuit 904, a plurality of filling pumps 906, 908 (for example one filling pump for each chamber pair), a return duct pump 910, a liquid sensor 912, an ultraviolet light source 914, and an electrical energy storage 916, for example a rechargeable battery or a power capacitor.

The control circuit 904 may include for example a control logic based on analogue and/or digital circuits. The control circuit 904 may include power amplifiers for driving the ultrasonic transducers 122-136. The control circuit 904 may include separate power amplifiers for each ultrasonic transducer 122-136 or may drive a group or all of the ultrasonic transducers 122-136 with a single power amplifier. The control logic 904 may be based on a microprocessor or a digital signal processor in combination with a computer program and memory for storing the computer program and data. The one or more power amplifiers may drive the ultrasonic transducers 122-136 at a frequency in a range of 1 to 2 MHz and may be tuneable with a resolution of a few kHz, for example with a resolution of 3 kHz. The driving waveform may be sinusoidal for electromagnetic compatibility (EMC) issues. Screened transducer mountings may allow easy servicing and reduce electromagnetic compatibility EMC issues.

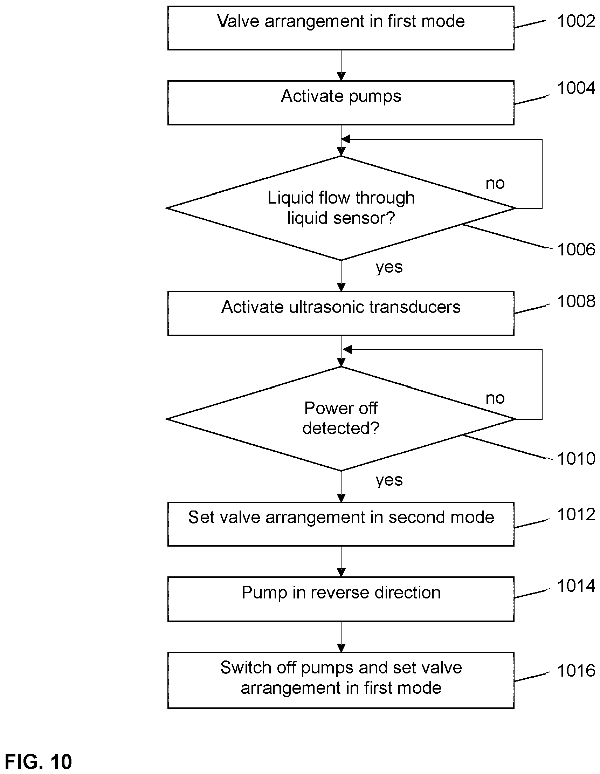

As depicted, the control circuit 904 is coupled to the ultrasonic transducers 122-136, the pumps 906-910, the liquid sensor 912, the ultraviolet light source 914 and a valve arrangement 918 at the reservoir 902. Operation of the haze generating device 100 under the control of the control circuit 904 may be as follows, see FIG. 10.

When the haze generating device 100 is installed at a venue, for example at a stage of a theatre or concert hall, the reservoir 902 may be filled with liquid via a filling inlet 920. At this point, the control circuit 904 may set the valve arrangement 918 to a first mode in which the valve arrangement 918 allows air to flow into the reservoir 902 but does not allow air to escape from the reservoir 902 (step 1002). The filling inlet 920 includes a tubing extending downwardly within the reservoir 902 such that the reservoir 902 can only be filled to a filling level 922 only and a certain volume 924 remains unfilled and cannot be filled since the air in this volume 924 cannot escape while the valve arrangement 918 is in the first mode. As this volume is not visible to an operator of the haze generating device 100, it is also referred to here as a "hidden volume". However, when the filling inlet 920 is closed with a lid, liquid can be taken from the reservoir 902 without creating a negative pressure, as the valve arrangement 918 allows air to flow into the reservoir 902. The reservoir 902 may have a volume of a few litres, for example 3 litres, without the hidden volume.

The operator may then switch on the haze generating device 100. The control circuit controls the filling pump 906 to pump liquid from the reservoir 902 into the chambers 108 and 110. See step 1004. Once the chambers 108 and 110 are filled to the desired filling level 802, any excess of liquid drains through the liquid outlet 706 of the chamber pair 108/110 and is returned to the reservoir 902. Additionally and in the same way, the control circuit 904 controls the filling pump 908 to pump liquid from the reservoir 902 into the chambers 106 and 112. The remaining chamber pairs 104/114 and 102/116 may be filled with additional (not shown) filling pumps. Such a scheme can be extended to any number of chambers or chamber pairs. In particular, such a scheme can be applied also to chambers which are not arranged in pairs but that are individual chambers with individual liquid inlets and liquid outlets. Any excess liquid from the chamber pairs may drain through a return duct 926 back to the reservoir 902. The return duct 926 may be provided with the return duct pump 910 to facilitate return flow of the liquid into the reservoir 902. Additionally, the return duct 926 may be provided with the liquid sensor 912 which indicates whether liquid is flowing through the return duct 926. If the liquid sensor 912 indicates a liquid flow in step 1006, it can be assumed that at least some chambers are filled to the required filling level 802 without any further need of sensor detection in the chambers. The pumps may be low noise diaphragm and/or peristaltic pumps for low noise operation.

Once the chambers are reliably filled with liquid, the ultrasonic transducers 122-136 may be activated to generate droplets resulting in aerosol in the chambers and haze output. See step 1008. As the liquid is continuously circulated from the reservoir 902 through the chambers 102-116 and back to the reservoir 902, the ultrasonic transducers 122-136 can be cooled by the large amount of liquid. Overheating of the ultrasonic transducers 122-136 may be avoided. A low temperature of the liquid can be maintained.

A wear algorithm may spread working hours across all the available ultrasonic transducers. For example, some of the ultrasonic transducers may be temporarily switched off while others are still working. The wear algorithm may vary which ultrasonic transducers are switched off to achieve equally distributed working hours for all the ultrasonic transducers.

The control circuit 904 may control each ultrasonic transducer 122-136 individually, i.e. for each ultrasonic transducer a corresponding amplifier may be provided. The amplifiers may use a "push-pull" driver with a current feed. Piezo transducers usually have an acoustic resonance and crystal/mechanical resonances. For example, each amplifier may be controlled with feedback from noise of the current of the assigned ultrasonic transducer to keep the transducer in the parallel resonance, for example to allow high efficiency operation. The noise may be created by the random nature of the water flow in the fountain from which the droplets emerge. This may lead to variations in the load on the amplifier, which may be measured as noise in the range of 1 to 10 kHz. This can be observed in situations where the transducer is dirty on the surface and still a fountain is created, but no droplets since the ultrasound does not coupled to the liquid. It has been found that the peak of the signal is actually not the optimal point for the haze generation. A maximum haze output may be achieved at a few kilohertz below the measured peak resonance. The wear levelling algorithm may spread working hours across all available transducers irrespective of the output level.

In the reservoir 902, the ultraviolet light source 914 may be controlled by the control circuit 904 to emit ultraviolet light into the liquid for disinfection and hygienic control. The ultraviolet light source 914 may emit ultraviolet light with a wavelength of about 280 nm. Again, as the liquid is continuously circulated, the entire amount of liquid is continuously disinfected.

Although not shown in FIG. 9, the haze generating device 100 may include a mixing device for automatically adding a concentrate or additive, for example glycol, to the liquid in the reservoir 902. In this case, the reservoir 902 may be filled with tap water. The amount of tap water filled into the reservoir 902 may be measured at the inlet 920 or entered via a user interface by the operator. An appropriate amount of concentrate or additive is then automatically added to the liquid, for example by a pump or under the control of a valve or a venturi injector in a duct between the reservoir 902 and the pump 906. As the liquid is continuously circulated, the concentrate or additive is mixed with the tap water such that a liquid with homogeneous concentration of the concentrate or additive is obtained. The tap water may be treated using polyphosphate crystals to prevent limescale formation on surfaces of the chambers 102-116 and the reservoir 902.

When the haze generating device 100 is turned off or disconnected from the power supply (step 1010), the control circuit 904 may control the valve arrangement 918 to operate in a second mode using energy from the energy storage device 916, see step 1012. In the second mode, the valve arrangement 918 allows air to escape from the reservoir 902 to the outside. At the same time, the control circuit 904 may control the filling pumps 906, 908 to pump in the reverse direction with energy from the energy storage device 916. See step 1014. Thus, the liquid from the chambers 102-116 is pumped back into the reservoir 902. The hidden volume 924 is configured to be large enough to hold the liquid from all of the chambers 102-116. For example, the chambers 102 to 116 may be configured such that each chamber contains 50 to 200 ml when it is filled to the filling level 802. For example, each chamber pair may be configured such that it contains 175 ml when it is filled to the filling level 802, and a corresponding hidden volume 924 may be at least 700 ml to cover the volume of four chamber pairs. The filling pumps 906, 908 may be controlled to pump in the reverse direction for a time sufficient to ensure that all chambers 102-116 are emptied. The control circuit 904 may then switch off the filling pumps 906, 908 and may control the valve arrangement 918 to operate in the first mode such that the reservoir 902 is completely sealed from the outside. See step 1016. In this state, the haze generating device 100 can be transported even in a tilted position without any leakage of the liquid from the reservoir 902 or any of the chambers 102-116.

The liquid circulation system including the pumps 906, 908, 912 may be used to introduce a cleaning solution from a separate reservoir or tank via a corresponding valve arrangement (not shown in FIG. 9) for automatic system cleaning. This may be done automatically at the request of the operator, or upon detection of the haze generating device 100 being switched off.

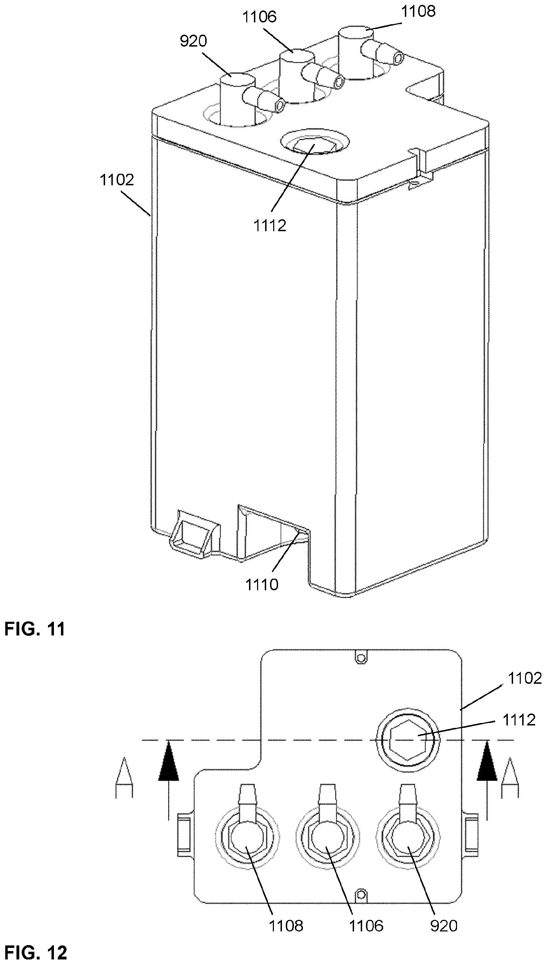

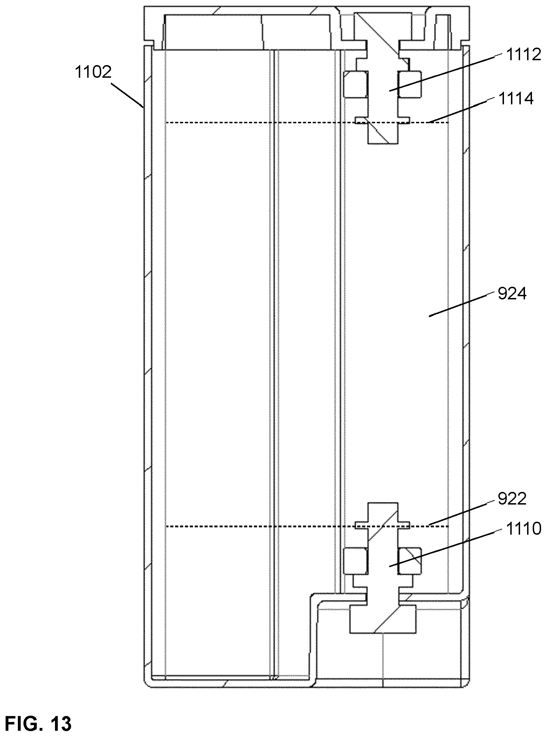

The above described reservoir 902 is only an example. A further exemplary reservoir 1102 will be described below in connection with FIGs. 11 to 13. The reservoir 1102 may be used in the same way as the reservoir 902 in the haze generating device 100. FIG. 11 shows a perspective view of the reservoir 1102, FIG. 12 shows a top view of the reservoir 1102 and FIG. 13 shows a sectional view of the reservoir 1102 along section A-A indicated in FIG. 12.

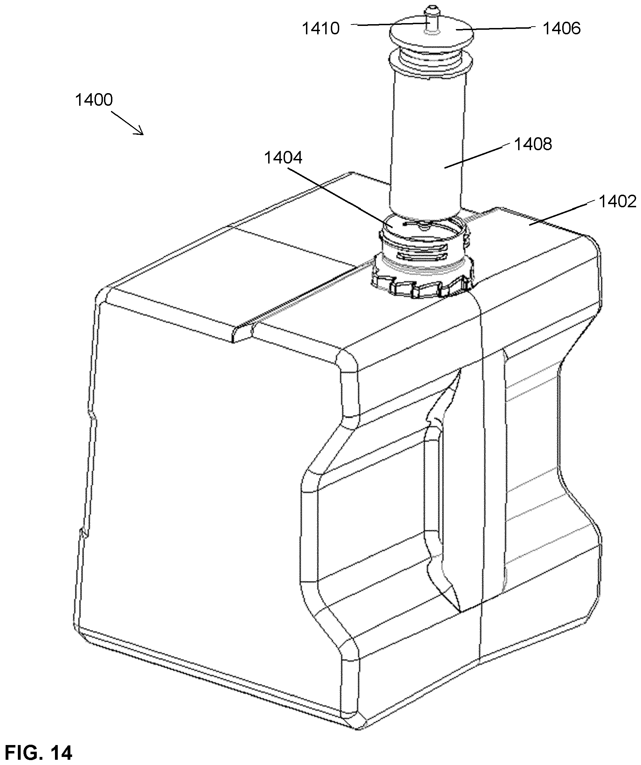

The reservoir 1102 has a filling inlet 920 through which water and concentrate to be used for haze generation may be filled into the reservoir 1102. The filling inlet 920 may be connected, for example via a corresponding water pump, to an external water tank having a volume of, for example, 5 to 10 litres. An exemplary water tank 1400 is shown in FIG. 14. The filling inlet 920 may additionally be connected, for example via a corresponding concentrate pump, to a concentrate tank having a volume of, for example, several hundred millilitres, for example 300 to 800 ml. Both the water pump and the concentrate pump may be controlled by the control circuit 904.

The reservoir 1102 has a liquid connector 1106 which may be connected to the chambers 106-116, for example via the pumps 906, 908 to the chamber pairs as is shown in FIG. 9. The reservoir 1102 has a return liquid connector 1108 which may be connected to the liquid outlets 706 of the chambers 106-116, for example via the return duct 926, the return duct pump 910 and the liquid sensor 912.

The reservoir 1102 may be provided with liquid level sensors 1110 and 1112. The liquid level sensor 1110 may be used to prevent the reservoir 1102 from being filled from the water tank and the concentrate tank to a level above the filling level 922. The liquid level sensor 1112 may be used to prevent the return of liquid from the chambers 106-116 above an overflow level 1114. The reservoir 1102 may have a total capacity of, for example, one litre when being filled to the overflow level 1114. A few hundred millilitres, for example 200 to 400 ml, may be required to fill the reservoir 1102 to the filling level 922. The predefined "hidden" volume 924 discussed above in connection with FIG. 9 corresponds to the volume between the filling level 922 and the overflow level 1114.

Although not shown in FIGs. 11 to 13, an ultraviolet light source may be provided in the reservoir 1102 and may be controlled by the control circuit 904 to emit ultraviolet light into the liquid for disinfection and hygienic control.

FIG. 14 shows details of the water tank 1400. The water tank 1400 may comprise a hollow body 1402, a lid 1406 and a filter 1408. The hollow body 1402 may have a volume in the range of for example 5 to 10 litres, for example the hollow body may have a volume of 6 litres. The hollow body has a filling opening 1404 which can be closed by the lid 1406. The filter 1408 is provided on a lower side of the lid 1406. An outlet connector 1410 is provided on an upper side of the lid 1406. The outlet connector 1410 may be connected to the filling inlet 920 of the reservoir 1102 via a pump. The hollow body 1402 of the water tank 1400 may be filled with tap water when the lid 1406 and the filter 1408 are removed. The filter 1408 at the lid 1406 is then inserted into the filling opening 1404 and the lid 1406 is secured to the filling opening 1404, for example by means of threads provided on the filling opening 1404 and the lid 1406. The tap water may be drawn from the hollow body 1402, the water passing through the filter 1408. The use of tap water may be problematic due to limescale which may leave a white coating on the inside of the components of the haze generating device 102, for example on the inside of the reservoir 902 and the chambers 102-116. The formation of the limescale may be at least partially avoided by the use of polyphosphates provided in the filter 1408. The filter 1408 may be a replenishable filter. As shown in FIG. 14, the filter may be located in the neck of the water tank 1400. As the water drawn from the hollow body 1402 passes through the filter 1408, a small amount of polyphosphate very slowly dissolves in the water stream and changes the reaction of the calcium in the water. As a result, the haze generating device 102 can be operated with tap water rather than demineralised water.

When the haze generating device 100 including the reservoir 1102 is installed at a venue, the filling inlet 920 may be connected to the water tank, for example to the outlet connector 1410 of the water tank 1400, and the concentrate tank via the water pump and the concentrate pump, respectively. The haze generating device 100 may be switched on and the control circuit 904 may activate the water pump and the concentrate pump to pump water and concentrate into the reservoir 1102 until the filling level 922 is reached. During further operation of the haze generating device 100, the control circuit 904 may continuously monitor the filling level using the liquid level sensor 1110 and may activate the water pump and the concentrate pump to pump water and concentrate into the reservoir 1102 whenever necessary to reach the filling level 922.

An operator may then control the control circuit 904 to start haze generation. The control circuit controls the filling pump 906 to pump liquid from the reservoir 1102 into the chambers 108 and 110. Once the chambers 108 and 110 are filled to the desired filling level 802, any excess of liquid drains through the liquid outlet 706 of the chamber pair 108/110 and is returned to the reservoir 1102. Additionally and in the same manner, the control circuit 904 controls the filling pump 908 to pump liquid from the reservoir 902 into the chambers 106 and 112. The remaining chamber pairs 104/114 and 102/116 may be filled with additional filling pumps. Any excess liquid from the chamber pairs may drain back to the reservoir 1102 through the return duct 926 and the return liquid connector 1108.

Once the chambers are reliably filled with liquid, the ultrasonic transducers 122-136 may be activated to generate droplets resulting in aerosol in the chambers and haze output. During haze generation, the control circuit 904 may continuously monitor the filling level using the liquid level sensor 1110 and may activate the water pump and the concentrate pump to pump water and concentrate into the reservoir 1102 to maintain the filling level 922.

When the haze generating device 100 is turned off or disconnected from the power supply, the control circuit 904 may control the filling pumps 906, 908 to pump in the reverse direction using energy from the energy storage device 916. In this way, the liquid from the chambers 102-116 is pumped back into the reservoir 1102. The hidden volume 924 is configured to be large enough to hold the liquid from all of the chambers 102-116. The filling pumps 906, 908 may be controlled to pump in the reverse direction for a time sufficient to ensure that all chambers 102-116 are emptied. The control circuit 904 may then switch off the filling pumps 906, 908. In this state, the haze generating device 100 can be transported even in a tilted position without any leakage of the liquid from the reservoir 902 or any of the chambers 102-116.

Generally, all terms used herein are to be interpreted according to their ordinary meaning in the relevant technical field, unless a different meaning is clearly given and/or is implied by the context in which they are used. All references to a/an/the element, apparatus, component, means, step, etc. are to be interpreted openly as referring to at least one instance of the element, apparatus, component, means, step, etc., unless explicitly stated otherwise. The steps of any method disclosed herein need not be performed in the exact order disclosed, unless a step is explicitly described as following or preceding another step and/or where it is implicit that a step must follow or precede another step. Any feature of any of the embodiments disclosed herein may be applied to any other embodiment, where appropriate. Likewise, any advantage of any of the embodiments may apply to any other embodiment and vice versa. Other objectives, features and advantages of the enclosed embodiments will be apparent from the description. As used herein, the term "configured to" means set up, organized, adapted, or arranged to operate in a particular way; the term is synonymous with "designed to". As used herein, the term "substantially" means nearly or essentially, but not necessarily completely; the term encompasses and accounts for mechanical or component value tolerances, measurement errors, random variation, and similar sources of inaccuracy.

The present invention may, of course, be carried out in ways other than those specifically set forth herein without departing from essential characteristics of the invention. The present embodiments are to be considered in all respects as illustrative and not restrictive, and all modifications coming within the meaning and equivalency range of the appended claims are intended to be embraced therein.

The following EXAMPLES define features which may be implemented in a haze generating device:
EXAMPLE 1: A haze generating device for generating aerosol from a liquid, the haze generating device comprising at least one chamber in which the aerosol is generated, wherein each chamber of the at least one chamber comprises:
   - a chamber housing including a bottom and a side wall extending from the bottom to a top of the chamber,
   - an ultrasonic transducer arranged at the bottom, and
   - a filter element arranged at the top of the chamber,

   wherein, in operation of the haze generating device, the chamber is at least partially filled with the liquid to a filling level such that at least an ultrasound emitting surface of the ultrasonic transducer is immersed in the liquid,
   wherein the chamber has a directed gas inlet at the side wall configured to direct a gas stream introduced into the chamber in a direction substantially parallel to a surface of the liquid in the chamber and substantially along the side wall,
   wherein the filter element comprises a plurality of guide surfaces configured to direct the gas stream out of the chamber in a direction substantially perpendicular to the surface of the liquid in the chamber.
EXAMPLE 2: The haze generating device of EXAMPLE 1, wherein each guide surface of the plurality of guide surfaces has a first surface portion extending perpendicular to the surface of the liquid in the chamber and a second surface portion extending angled from the first surface portion toward the surface of the liquid in the chamber.
EXAMPLE 3: The haze generating device of EXAMPLE 2, wherein the second surface portion extends in an angle in a range of 90 to 110 degrees, preferably 100 degrees, downwardly from the first surface portion.
EXAMPLE 4: The haze generating device of EXAMPLE 2 or EXAMPLE 3, wherein a length of extension of the second surface portion away from the first surface portion is in a range of 20% to 150%, preferable 100%, of the length of extension of the first surface portion perpendicular to the surface of the liquid in the chamber.
EXAMPLE 5: The haze generating device of any one of EXAMPLES 2-4, wherein the first surface portions of the plurality of guide surfaces are arranged parallel to each other, and/or the second surface portions of the plurality of guide surfaces are arranged parallel to each other.
EXAMPLE 6: The haze generating device of any one of EXAMPLES 2-5, wherein the first and second surface portions are each planar.
EXAMPLE 7: The haze generating device of any one of EXAMPLES 2-6, wherein the second surface portion extends from an edge of the first surface portion.
EXAMPLE 8: A haze generating device for generating aerosol from a liquid, the haze generating device comprising
   - at least one chamber in which the aerosol is generated, wherein each chamber of the at least one chamber comprises an ultrasonic transducer,
   - a reservoir for the liquid,
   - a pump coupled between the reservoir and the chamber for pumping the liquid from the reservoir into the chamber, and
   - a return duct connecting a liquid outlet of the chamber and the reservoir,
   wherein the liquid outlet is arranged in a level corresponding to the filling level.
EXAMPLE 9: The haze generating device of EXAMPLE 8, wherein the haze generating device comprises at least two chambers, wherein each chamber of the at least two chambers has an individual liquid outlet.
EXAMPLE 10. The haze generating device of EXAMPLE 8, wherein the haze generating device comprises a plurality of groups of chambers, wherein each group of chambers is fluidically separated from the other groups of chambers, wherein each group of chambers has an individual liquid outlet.
EXAMPLE 11. The haze generating device of EXAMPLE 10, wherein each group of chambers comprises two chambers.
EXAMPLE 12. The haze generating device of any one of EXAMPLES 8-11, wherein the return duct comprises a sensor for monitoring a return flow of the liquid from the chamber to the reservoir.
EXAMPLE 13. The haze generating device of EXAMPLE 12, wherein the return duct comprises a further pump for pumping the liquid from the chamber into the reservoir.
EXAMPLE 14. The haze generating device of EXAMPLE 13, wherein the further pump is controlled depending on a sensor value from the sensor.
EXAMPLE 15. The haze generating device of any one of EXAMPLES 8-14, wherein the haze generating device further comprises an ultraviolet light source emitting ultraviolet light into the liquid in the reservoir.
EXAMPLE 16: A haze generating device for generating aerosol from a liquid, the haze generating device comprising
   - at least one chamber in which the aerosol is generated, wherein each chamber of the at least one chamber comprises an ultrasonic transducer,
   - a reservoir for the liquid, and
   - a pump coupled between the reservoir and the chamber for pumping the liquid from the reservoir into the chamber,
   wherein a filling inlet of the reservoir for filling the liquid into the reservoir has a level control for ensuring that a predefined volume within the reservoir cannot be filled with the liquid via the filling inlet.
EXAMPLE 17. The haze generating device of EXAMPLE 16, wherein the predefined volume corresponds to a sum of volumes of the liquid in the plurality of chambers in operation of the haze generating device.
EXAMPLE 18. The haze generating device of any one of EXAMPLE 16 or EXAMPLE 17, wherein the haze generating device comprises a control circuit configured to operate the pump in the reverse direction for pumping the liquid from the chamber into the reservoir.
EXAMPLE 19. The haze generating device of EXAMPLE 18, wherein the control circuit comprises an electrical energy storage means for operating the pump in the reverse direction with electrical energy from the electrical energy storage means upon detection of an external power supply being turned off.
EXAMPLE 20. The haze generating device of any one of EXAMPLES 8-19, wherein the haze generating device comprises an additive tank and a mixing device coupled to the additive tank and the reservoir, wherein the mixing device is configured to add an additive contained in the additive tank to the liquid in the reservoir.
EXAMPLE 21. The haze generating device of any one of the preceding EXAMPLES, wherein the ultrasound emitting surface of the ultrasonic transducer is inclined between 15 and 22 degrees with respect to the surface of the liquid in the chamber.
EXAMPLE 22. The haze generating device of any one of the preceding EXAMPLES, wherein the haze generating device comprises eight chambers arranged in an array of four by two chambers.
EXAMPLE 23. The haze generating device of any one of the preceding EXAMPLES, wherein the ultrasonic transducer is driven by a control circuit with an electrical signal with sinusoidal waveform.
EXAMPLE 24. The haze generating device of any preceding EXAMPLES, wherein the haze generating device comprises at least two chambers, wherein a control circuit controls operation of the ultrasonic transducers of the at least two chambers to level operating times of the ultrasonic transducers.
EXAMPLE 25. The haze generating device of any one of the preceding EXAMPLES, wherein the ultrasonic transducer is controlled by a control circuit at parallel resonance.
EXAMPLE 26. The haze generating device of any one of the preceding EXAMPLES, wherein the ultrasonic transducer is controlled by a control circuit depending on an electrical noise signal determined at an electrical current driving the ultrasonic transducer.

## Claims

1. A haze generating device for generating aerosol from a liquid, the haze generating device (100) comprising at least one chamber (102-116) in which the aerosol is generated, wherein each chamber (102-116) of the at least one chamber (102-116) comprises:
- a chamber housing including a bottom and a side wall extending from the bottom to a top of the chamber (102-116),
- an ultrasonic transducer (122-136) arranged at the bottom, and
- a filter element (502, 504) arranged at the top of the chamber (102-116),
wherein, in operation of the haze generating device (100), the chamber (102-116) is at least partially filled with the liquid to a filling level (802) such that at least an ultrasound emitting surface (804, 806) of the ultrasonic transducer (122-136) is immersed in the liquid,
wherein the chamber (102-116) has a directed gas inlet (808, 810) at the side wall configured to direct a gas stream introduced into the chamber (102-116) in a direction substantially parallel (812, 814) to a surface of the liquid in the chamber (102-116) and substantially along the side wall,
wherein the filter element (502, 504) comprises a plurality of guide surfaces (702) configured to direct the gas stream out of the chamber (102-116) in a direction substantially perpendicular (828, 830) to the surface of the liquid in the chamber (102-116).

2. The haze generating device of claim 1, wherein each guide surface of the plurality of guide surfaces (702) has a first surface portion (824) extending perpendicular to the surface of the liquid in the chamber (102-116) and a second surface portion (826) extending angled from the first surface portion (824) toward the surface of the liquid in the chamber (102-116).

3. The haze generating device of claim 2, wherein the second surface portion (826) extends in an angle in a range of 90 to 110 degrees, preferably 100 degrees, downwardly from the first surface portion (824).

4. The haze generating device of claim 2 or claim 3, wherein the first surface portions (824) of the plurality of guide surfaces (702) are arranged parallel to each other, and/or
the second surface portions (826) of the plurality of guide surfaces (702) are arranged parallel to each other.

5. The haze generating device of any preceding claims, wherein the haze generating device (100) further comprises:
- a reservoir (902, 1102) for the liquid,
- a pump (906, 908) coupled between the reservoir (902, 1102) and the chamber (102-116) for pumping the liquid from the reservoir (902, 1102) into the chamber (102-116), and
- a return duct (926) connecting a liquid outlet (706) of the chamber (102-116) and the reservoir (902, 1102),
wherein the liquid outlet (706) is arranged in a level corresponding to the filling level (802).

6. The haze generating device of claim 5, wherein the haze generating device (100) comprises at least two chambers, wherein each chamber of the at least two chambers has an individual liquid outlet.

7. The haze generating device of claim 5, wherein the haze generating device (100) comprises a plurality of groups of chambers (102/116, 104/114, 106/112, 108/110), wherein each group of chambers is fluidically separated from the other groups of chambers, wherein each group of chambers has an individual liquid outlet (706).

8. The haze generating device of any one of claims 5-7, wherein the return duct (926) comprises a sensor (912) for monitoring a return flow of the liquid from the chamber (102-116) to the reservoir (902, 1102).

9. The haze generating device of any one of claims 5-8, wherein the return duct (926) comprises a further pump (910) for pumping the liquid from the chamber (102-116) into the reservoir (902, 1102).

10. The haze generating device of any one of claims 5-9, wherein the haze generating device (100) further comprises an ultraviolet light source (914) emitting ultraviolet light into the liquid in the reservoir (902, 1102).

11. The haze generating device of any one of claims 5-10, wherein a filling inlet (920) of the reservoir (902, 1102) for filling the liquid into the reservoir (902, 1102) has a level control for ensuring that a predefined volume (924) within the reservoir (902, 1102) cannot be filled with the liquid via the filling inlet (920).

12. The haze generating device of any one of claims 5-11, wherein the haze generating device (100) comprises a control circuit (904) configured to operate the pump (906, 908) in the reverse direction for pumping the liquid from the chamber (102-116) into the reservoir (902, 1102).

13. The haze generating device of claim 12, wherein the control circuit (904) comprises an electrical energy storage means (916) for operating the pump (906, 908) in the reverse direction with electrical energy from the electrical energy storage means (916) upon detection of an external power supply being turned off.

14. The haze generating device of any one of claims 5-13, wherein the haze generating device (100) comprises an additive tank and a mixing device coupled to the additive tank and the reservoir (902, 1102), wherein the mixing device is configured to add an additive contained in the additive tank to the liquid in the reservoir (902, 1102).

15. The haze generating device of any one of the preceding claims, wherein the ultrasonic transducer (122-136) is driven by a control circuit (904) with an electrical signal with sinusoidal waveform and/or at parallel resonance and/or depending on an electrical noise signal determined at the electrical current driving the ultrasonic transducer (122-136).
